# EUROPEAN PATENT APPLICATION

(11) **EP 4 317 161 A1**
(43) Date of publication of application: **07.02.2024**
(21) Application number: 22775281.3
(22) Date of filing: 15.03.2022
(51) Int. Cl.: C07D 487/04, C01B 39/48

(54) **PYRROLIDINE DERIVATIVE, PRODUCTION METHOD THEREFOR, MSE-TYPE ZEOLITE, AND PRODUCTION METHOD THEREFOR**

(30) Priority: 25.03.2021 JP 2021051555
(71) Applicant: Mitsui Mining & Smelting Co., Ltd., Shinagawa-ku Tokyo 141-8584 (JP)
(72) Inventor: HAYASHI Katsuhiko, Ageo-shi, Saitama 362-0021 (JP); TAMURA Kenji, Ageo-shi, Saitama 362-0021 (JP); MOGI Satoshi, Ageo-shi, Saitama 362-0021 (JP); TAKAHASHI Susumu, Ageo-shi, Saitama 362-0021 (JP); GOTO Hidekazu, Ageo-shi, Saitama 362-0025 (JP)
(74) Representative: Novagraaf Technologies
(86) International application number: PCT/JP2022/011624
(87) International publication number: WO 2022/202491

(57) **Abstract**

Provided are: a pyrrolidine derivative containing quaternary ammonium cations having a bicyclo framework that can be used as an organic structure directing agent; a method for producing the same; an MSE-type zeolite; and a method for producing the same.

The pyrrolidine derivative containing quaternary ammonium cations having a bicyclo framework has, in an X-ray diffraction spectrum, a highest peak intensity I in a diffraction angle (2θ) range of 14.0° to 16.0° and a highest peak intensity II in a diffraction angle (2θ) range of 17.0° to 19.0°, wherein a peak intensity ratio (I/II) of the peak intensity I to the peak intensity II is in a range of 0.4 or more and 1.5 or less.

## Description

### TECHNICAL FIELD

The present invention relates to a pyrrolidine derivative, a method for producing the same, an MSE-type zeolite, and a method for producing the same.

### BACKGROUND ART

Exhaust gases emitted from internal combustion engines of automobiles and other vehicles using gasoline as fuel contain harmful components such as hydrocarbons (HCs), and to treat such harmful components, exhaust gas purifying catalysts are used.

Exhaust gas purifying catalysts are known to be inefficient in treating exhaust gases in low temperature environments such as in cold-start conditions of internal combustion engines. To improve the efficiency of exhaust gas purification in cold-start conditions, there are cold-start catalysts that include zeolite catalysts containing base metals, precious metals, and zeolites. For example, BEA-type zeolites, MFI-type zeolites, and CHA-type zeolites are known as zeolites included in cold-start catalysts, and an MSE-type zeolite and its production method are disclosed in Patent Literature 1.

### CITATION LIST

### PATENT LITERATURE

Patent Literature 1: Japanese Translation of PCT International Application Publication No. 2002-535227

### SUMMARY OF THE INVENTION

### PROBLEM TO BE SOLVED BY THE INVENTION

Exhaust gas purifying catalysts including cold-start catalysts are required to have the heat resistance of the aforementioned zeolite serving as a hydrocarbon adsorbent such that hydrocarbons in the exhaust gas are stored in a low-temperature environment at the start of an internal combustion engine, and the stored hydrocarbons are released after the catalyst is sufficiently activated by the inflow of high-temperature exhaust gas.

The production method described in, for example, Patent Literature 1 uses a crystallization method, which yields a substance that serves as an organic structure directing agent (OSDA) for zeolites, but also forms a large amount of hardly-water-soluble impurities. An organic structure directing agent contaminated with hardly-water-soluble impurities can be easily purified by adding water and filtering, but there are problems such as the fact that adding water increases the total volume during transportation, which increases transportation costs, requires a large storage space, and makes long-term storage difficult. It is also difficult to separately use hardly-water-soluble impurities and an organic structure directing agent by forming into powder form. When a zeolite is produced using an organic structure directing agent containing a large amount of hardly-water-soluble impurities, the produced zeolite contains a large amount of an MSE-type crystal phase and crystal phases other than the MSE-type crystal phase (e.g., MTW phase), and the crystal phases other than the MSE-type crystal phase cause defects, resulting in low heat resistance.

Accordingly, an object of the present invention is to provide, in order to obtain a hydrocarbon adsorbent having excellent heat resistance, a pyrrolidine derivative containing quaternary ammonium cations having a bicyclo framework that can be used as an organic structure directing agent for an MSE-type zeolite, a method for producing the same, an MSE-type zeolite, and a method for producing the same.

### MEANS FOR SOLVING PROBLEM

The present invention provides a pyrrolidine derivative containing quaternary ammonium cations having a bicyclo framework, and having an X-ray diffraction spectrum with a highest peak intensity I in a diffraction angle (2θ) range of 14.0° to 16.0° and a highest peak intensity II in a diffraction angle (2θ) range of 17.0° to 19.0°, wherein a peak intensity ratio (I/II) of the peak intensity I to the peak intensity II is in a range of 0.4 or more and 1.5 or less.

The present invention also provides an MSE-type zeolite having an X-ray diffraction spectrum with a highest peak intensity A in a diffraction angle (2θ) range of 21.0° to 22.0° representing the (420) plane, and a highest peak intensity B in a diffraction angle (2θ) range of 22.6° to 23.6° representing the (115) plane, wherein a peak intensity ratio (A/B) of the peak intensity A to the peak intensity B is in a range of 1.25 or more and 2.50 or less.

### EFFECT OF THE INVENTION

The pyrrolidine derivative containing quaternary ammonium cations having a bicyclo framework provided by the present invention can be used as an organic structure directing agent for an MSE-type zeolite containing few hardly-water-soluble impurities, containing a large amount of an MSE-type crystal phase, and suppressing the formation of crystal phases other than the MSE-type crystal phase. The MSE-type zeolite provided by the present invention has a low content of crystal phases other than the MSE-type crystal phase, which cause defects, and has excellent heat resistance.

### BRIEF DESCRIPTION OF DRAWINGS

FIG. 1 shows an X-ray diffraction spectrum of a pyrrolidine derivative 2 in Example 3.
FIG. 2 shows an X-ray diffraction spectrum of a pyrrolidine derivative 2 in Comparative Example 1.
FIG. 3 shows an SEM micrograph of a pyrrolidine derivative 2 in Example 2.
FIG. 4 shows an SEM micrograph of a pyrrolidine derivative 2 in Comparative Example 1.
FIG. 5 shows an X-ray diffraction spectrum of an MSE-type zeolite (with OSDA) in Example 3.
FIG. 6 shows an X-ray diffraction spectrum of an MSE-type zeolite (with OSDA) in Comparative Example 1.
FIG. 7 shows an X-ray diffraction spectrum of an MSE-type zeolite (without OSDA) in Example 3.
FIG. 8 shows an X-ray diffraction spectrum of an MSE-type zeolite (without OSDA) in Comparative Example 1.

### MODE(S) FOR CARRYING OUT THE INVENTION

Next, the present invention will be described based on exemplary embodiments. However, the present invention is not limited to the embodiments described below.

### Pyrrolidine Derivative

An exemplary embodiment of the present invention provides a pyrrolidine derivative containing quaternary ammonium cations having a bicyclo framework, and having an X-ray diffraction spectrum with a highest peak intensity I in a diffraction angle (2θ) range of 14.0° to 16.0° and a highest peak intensity II in a diffraction angle (2θ) range of 17.0° to 19.0°, wherein a peak intensity ratio (I/II) of the peak intensity I to the peak intensity II is in a range of 0.4 or more and 1.5 or less.

### Quaternary Ammonium Cations Having Bicyclo Framework

The quaternary ammonium cations having a bicyclo framework preferably contain N,N,N',N'-tetraalkylbicyclo[2.2.2]oct-7-ene-2,3;5,6-dipyrrolidinium dication represented by the following formula (1) and/or N,N,N',N'-tetraalkylbicyclo[2.2.2]octane-2,3;5,6-dipyrrolidinium dication represented by the following formula (2).

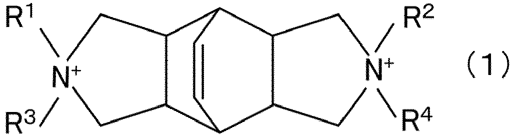

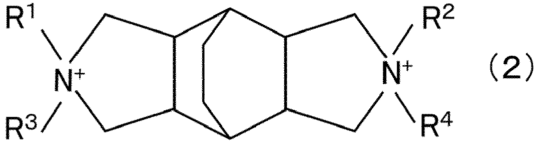

In the formula (1) or the formula (2), R¹, R², R³, and R⁴ each independently represent a linear or branched alkyl group having 1 to 4 carbon atoms. In the formula (1) or the formula (2), R¹, R², R³, and R⁴ each independently include a methyl group, an ethyl group, an n-propyl group, an isopropyl group, an n-butyl group, a sec-butyl group, an isobutyl group, and a tert-butyl group. In the formula (1) or the formula (2), R¹, R², R³, and R⁴ are each preferably an ethyl group.

The pyrrolidine derivative may be a pyrrolidinium salt represented by the following formula (3), or may be a pyrrolidinium salt represented by the following formula (4).

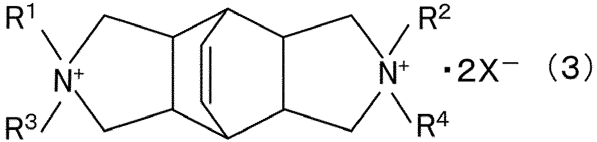

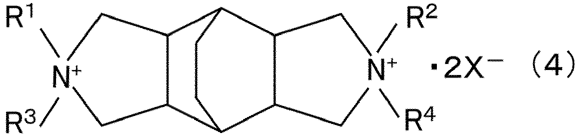

In the formula (3) or the formula (4), R¹, R², R³, and R⁴ each independently have the same meaning as in the formula (1) or the formula (2), and X represents at least one halogen atom selected from the group consisting of bromine and iodine.

Examples of the pyrrolidinium salt, as the pyrrolidine derivative, include N,N,N',N'-tetraalkylbicyclo[2.2.2]oct-7-ene-2,3;5,6-dipyrrolidinium iodide, N,N,N',N'-tetraalkylbicyclo[2.2.2]octane-2,3;5,6-dipyrrolidinium iodide, N,N,N',N'-tetraalkylbicyclo[2.2.2]oct-7-ene-2,3;5,6-dipyrrolidinium bromide, and N,N,N',N'-tetraalkylbicyclo[2.2.2]octane-2,3;5,6-dipyrrolidinium bromide.

The pyrrolidine derivative containing quaternary ammonium cations having a bicyclo framework (hereinafter also referred to as "pyrrolidine derivative" in the present specification) preferably has an X-ray diffraction spectrum with a highest peak intensity I in a diffraction angle (2θ) range of 14.0° to 16.0° and a highest peak intensity II in a diffraction angle (2θ) range of 17.0° to 19.0°. Further, the pyrrolidine derivative preferably has a peak intensity ratio (I/II) of the peak intensity I to the peak intensity II in a range of 0.4 or more and 1.5 or less, more preferably in a range of 0.45 or more and 1.0 or less, and particularly preferably in a range of 0.45 or more and 0.75 or less.

When the peak intensity ratio (I/II) falls within the range of 0.4 or more and 1.5 or less, it can be said that hardly-water-soluble impurities are highly dispersed in the pyrrolidine derivative, and by using a pyrrolidine derivative having a peak intensity ratio (I/II) within such a range to produce an MSE-type zeolite, precipitation of crystal phases other than the MSE-type thereof can be suppressed starting from the hardly-water-soluble impurities present in the pyrrolidine derivative to obtain an MSE-type zeolite having a highly pure MSE-type crystal phase. Such an MSE-type zeolite having a highly pure MSE-type crystal phase has excellent heat resistance.

On the other hand, when the peak intensity ratio (I/II) is less than 0.4 or more than 1.5, it can be said that the pyrrolidine derivative is not formed in the first place, or hardly-water-soluble impurities are not highly dispersed in the pyrrolidine derivative, and by using a pyrrolidine derivative having a peak intensity ratio (VII) of less than 0.4 or more than 1.5 to produce an MSE-type zeolite, a zeolite having an MSE-type crystal phase can be obtained, and at the same time, an MSE-type zeolite having crystal phases other than the MSE-type crystal phase starting from the hardly-water-soluble impurities present in the pyrrolidine derivative can be obtained.

The X-ray diffraction spectrum of the pyrrolidine derivative can be measured using an X-ray diffractometer (e.g., model number: RINT-TTR III, manufactured by Rigaku Corp.).

The pyrrolidine derivative preferably has an X-ray diffraction spectrum of α-aluminum oxide with a peak intensity ratio (I/α) of the peak intensity I to a peak intensity α of the (116) plane of 0.1 or more, more preferably 0.15 or more. In addition, the peak intensity ratio (I/α) is preferably 0.9 or less, more preferably 0.5 or less, and particularly preferably 0.35 or less. In the present specification, α-aluminum oxide (α-alumina) refers to X-ray diffraction standard sample 674a distributed by the National Institute of Standards and Technology (NIST). When the peak intensity ratio (I/α) falls within such a range, the pyrrolidine derivative contains few hardly-water-soluble impurities and easily forms a highly pure MSE-type crystal phase.

The pyrrolidine derivative preferably has a peak intensity ratio (II/α) of the peak intensity II to the peak intensity α of 0.3 or more, more preferably 0.31 or more.

In addition, the peak intensity ratio (II/α) is preferably 0.5 or less, and more preferably 0.45 or less. When the peak intensity ratio (II/α) falls within such a range, the pyrrolidine derivative containing quaternary ammonium cations having a bicyclo framework contains few hardly-water-soluble impurities and easily forms a highly pure MSE-type crystal phase.

The pyrrolidine derivative preferably has a crystallite size determined from the peak having the peak intensity I in a range of 45 nm or more and 120 nm or less. The pyrrolidine derivative of the present invention produced by the production method described below is produced in a short period of time while incorporating hardly-water-soluble impurities, which tends to result in small crystallite size and highly dispersed hardly-water-soluble impurities. By using such a pyrrolidine derivative to produce an MSE-type zeolite, the hardly-water-soluble impurities can be sufficiently dissolved in the zeolite synthesis raw material to obtain an MSE-type zeolite having few crystalline phases other than the MSE-type crystalline phases starting from the hardly-water-soluble impurities. The crystallite size determined from the peak having the peak intensity I of the pyrrolidine derivative may preferably be in a range of 48 nm or more and 105 nm or less. The crystallite size can be measured, for example, by the method described below.

The pyrrolidine derivative preferably has a BET specific surface area of 2.0 m²/g or more, and it may be 3.0 m²/g or more, may be 4.0 m²/g or more, or may be 10.0 m²/g or more. The pyrrolidine derivative of the present invention produced by the production method described below is produced in a short period of time while incorporating hardly-water-soluble impurities, which tends to result in a BET specific surface area of 2.0 m²/g or more and highly dispersed hardly-water-soluble impurities. By using such a pyrrolidine derivative to produce an MSE-type zeolite, the hardly-water-soluble impurities can be sufficiently dissolved in the zeolite synthesis raw material to obtain an MSE-type zeolite having few crystalline phases other than the MSE-type crystalline phases starting from the hardly-water-soluble impurities. The BET specific surface area can be measured, for example, by the method described below.

The pyrrolidine derivative preferably has a primary particle diameter of 3.0 µm or less, which can be measured by a scanning electron microscope (SEM) micrograph of the pyrrolidine derivative, and it may be in a range of 0.3 µm or more and 1.5 µm or less. The pyrrolidine derivative of the present invention produced by the production method described below is produced in a short period of time while incorporating hardly-water-soluble impurities, which tends to result in a primary particle diameter of 3.0 µm or less measured by the SEM micrograph of the particles and highly dispersed hardly-water-soluble impurities. By using such a pyrrolidine derivative to produce an MSE-type zeolite, the hardly-water-soluble impurities can be sufficiently dissolved in the zeolite synthesis raw material to obtain an MSE-type zeolite having few crystalline phases other than the MSE-type crystalline phases starting from the hardly-water-soluble impurities. The primary particle diameter of the pyrrolidine derivative can be determined by observing the pyrrolidine derivative particles at a magnification of 30,000 times using a field emission scanning electron microscope (FE-SEM), and calculating an average value of Feret's diameters of 50 pyrrolidine derivative particles arbitrarily selected from the field of view.

### Method for Producing Pyrrolidine Derivative

A method for producing a pyrrolidine derivative includes a first step of preparing N,N'-dialkylbicyclo[2.2.2]oct-7-ene-2,3;5,6-tetracarboxydiimide or N,N'-dialkylbicyclo[2.2.2]octane-2,3;5,6-tetracarboxydiimide. The method also includes a second step of reacting the N,N'-dialkylbicyclo[2.2.2]oct-7-ene-2,3;5,6-tetracarboxydiimide or the N,N'-dialkylbicyclo[2.2.2]octane-2,3;5,6-tetracarboxydiimide with a reducing agent to obtain N,N'-dialkylbicyclo[2.2.2]oct-7-ene-2,3;5,6-dipyrrolidine and/or N,N'-dialkylbicyclo[2.2.2]octane-2,3;5,6-dipyrrolidine. The method also includes a third step of reacting the N,N'-dialkylbicyclo[2.2.2]oct-7-ene-2,3;5,6-dipyrrolidine and/or the N,N'-dialkylbicyclo[2.2.2]octane-2,3;5,6-dipyrrolidine with an alkyl halide to precipitate an N,N,N',N'-tetraalkylbicyclo[2.2.2]oct-7-ene-2,3;5,6-dipyrrolidinium salt and/or an N,N,N',N'-tetraalkylbicyclo[2.2.2]octane-2,3;5,6-dipyrrolidinium salt, thereby obtaining a pyrrolidine derivative.

In addition, the method for producing a pyrrolidine derivative preferably satisfies at least one of the following condition (1) or (2). The pyrrolidine derivative obtained after the second step and before the third step is also referred to as "pyrrolidine derivative 1". The pyrrolidinium salt obtained after the third step is also referred to as "pyrrolidine derivative 2".

Condition (1): In the second step, the reducing agent contains at least one selected from the group consisting of sodium bis(2-methoxyethoxy)aluminum hydride, lithium triethylborohydride, diisobutylaluminum hydride, diborane, lithium borohydride, and sodium borohydride.

Condition (2): In the third step, the alkyl halide is reacted in the presence of an organic solvent in a molar ratio of 13 times or less relative to the N,N'-dialkylbicyclo[2.2.2]oct-7-ene-2,3;5,6-dipyrrolidine and/or the N,N'-dialkylbicyclo[2.2.2]octane-2,3;5,6-dipyrrolidine.

As a first example of the method for producing a pyrrolidine derivative, a scheme (S 1) for obtaining a pyrrolidine derivative is described below. The scheme (S 1) includes the following first to third steps.

In a first step, N,N'-bicyclo[2.2.2]oct-7-ene-2,3;5,6-tetracarboxylic dianhydride (a) and an alkylamine are refluxed in the presence of water to obtain N,N'-dialkylbicyclo[2.2.2]oct-7-ene-2,3;5,6-tetracarboxydiimide (b).

In a second step, the resulting N,N'-dialkylbicyclo[2.2.2]oct-7-ene-2,3;5,6-tetracarboxydiimide (b) is reacted with a reducing agent in the condition (1) or with a reducing agent other than in the condition (1) to obtain N,N'-dialkylbicyclo[2.2.2]oct-7-ene-2,3;5,6-dipyrrolidine (c).

In a third step, the resulting N,N'-dialkylbicyclo[2.2.2]oct-7-ene-2,3;5,6-dipyrrolidine (c) is reacted with an alkyl halide under the condition (2) or under conditions other than the condition (2) to obtain an N,N'-tetraalkylbicyclo[2.2.2]oct-7-ene-2,3;5,6-dipyrrolidinium salt (d).

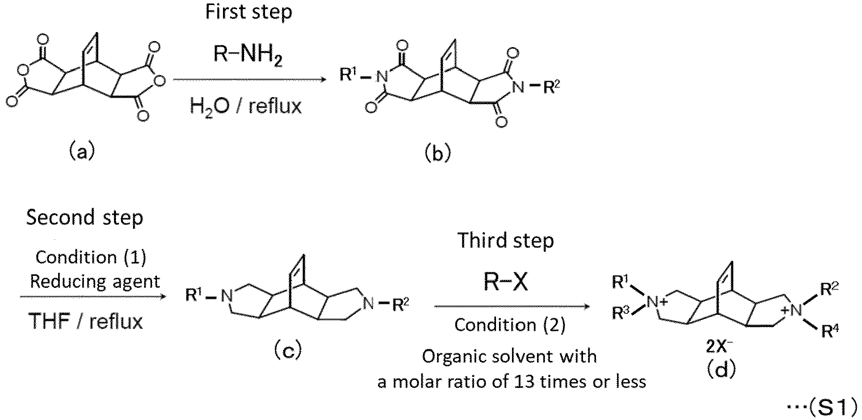

Each notation in the scheme (S1), and in a scheme (S2) and a scheme (S3) described below has the following meaning. The "R-NH₂" represents an alkylamine. The "R-X" represents an alkyl halide. The "R" represents a linear or branched alkyl group having 1 to 4 carbon atoms. The "R", "R¹", "R²", "R³", and "R⁴" each independently have the same meaning as R¹, R², R³, and R⁴ in the formulas (1) to (4). The "X" has the same meaning as X in the formula (3) or (4). The "THF" represents tetrahydrofuran. The "reflux" indicates refluxing.

Next, as a second example of the method for producing a pyrrolidine derivative, a scheme (S2) for obtaining a pyrrolidine derivative is described below. The scheme (S2) includes the following first to third steps.

In a first step, N,N'-bicyclo[2.2.2]octane-2,3;5,6-tetracarboxylic dianhydride (e) and an alkylamine are refluxed in the presence of water to obtain N,N'-dialkylbicyclo[2.2.2]octane-2,3;5,6-tetracarboxydiimide (f).

In a second step, the resulting N,N'-dialkylbicyclo[2.2.2]octane-2,3;5,6-tetracarboxydiimide (f) is reacted with a reducing agent in the condition (1) or with a reducing agent other than in the condition (1) to obtain N,N'-dialkylbicyclo[2.2.2]octane-2,3;5,6-dipyrrolidine (g).

In a third step, the resulting N,N'-dialkylbicyclo[2.2.2]octane-2,3;5,6-dipyrrolidine (g) is reacted with an alkyl halide under the condition (2) or under conditions other than the condition (2) to obtain an N,N'-tetraalkylbicyclo[2.2.2]octane-2,3;5,6-dipyrrolidinium salt (h).

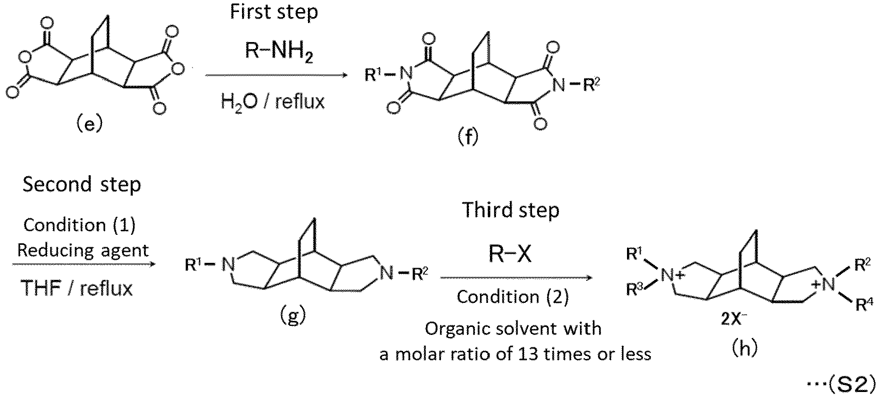

Next, as a third example of the method for producing a pyrrolidine derivative, a scheme (S3) for obtaining a pyrrolidine derivative is described below. The scheme (S3) includes the following first to third steps.

In a first step, N,N'-bicyclo[2.2.2]oct-7-ene-2,3;5,6-tetracarboxylic dianhydride (a) and an alkylamine are refluxed in the presence of water to obtain N,N'-dialkylbicyclo[2.2.2]oct-7-ene-2,3;5,6-tetracarboxydiimide (b).

In a second step, the resulting N,N'-dialkylbicyclo[2.2.2]oct-7-ene-2,3;5,6-tetracarboxydiimide (b) can be reduced with hydrogen under pressurized conditions of, e.g., 100°C to 300°C and 3 to 30 MPa in the presence of a base metal catalyst such as copper-chromium or Raney nickel, or a precious metal catalyst having a precious metal supported on silica, alumina, or a carbon support to obtain N,N'-dialkylbicyclo[2.2.2]octane-2,3;5,6-dipyrrolidine (g). Examples of the precious metal include at least one selected from the group consisting of Pt, Pd, Rh, Ir, and Ru. The hydrogen reduction in the second step may be performed under pressure conditions of 150°C and 5 MPa, for example, when a precious metal catalyst is used.

In a third step, the resulting N,N'-dialkylbicyclo[2.2.2]octane-2,3;5,6-dipyrrolidine (g) is reacted with an alkyl halide under the condition (2) or under conditions other than the condition (2) to obtain an N,N'-tetraalkylbicyclo[2.2.2]octane-2,3;5,6-dipyrrolidinium salt (h).

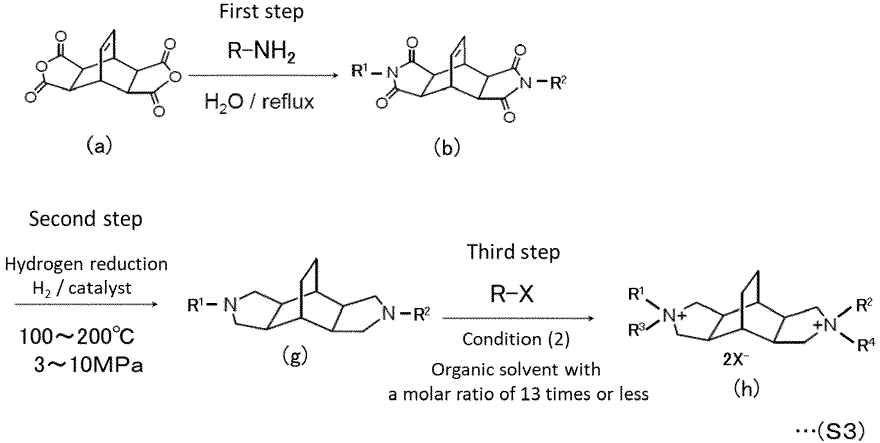

The method of producing a pyrrolidine derivative may satisfy either the condition (1) in the second step or the condition (2) in the third step, or it may satisfy both the condition (1) in the second step and the condition (2) in the third step.

### First Step

In the first step, an alkylamine aqueous solution is charged into a reaction vessel in which the alkylamine aqueous solution is obtained by dissolving an alkylamine having a molar ratio of 10 times or more and 30 times or less relative to N,N'-dialkylbicyclo[2.2.2]oct-7-ene-2,3;5,6-tetracarboxylic dianhydride or N,N'-dialkylbicyclo[2.2.2]octane-2,3;5,6-tetracarboxylic dianhydride (hereinafter also referred to as "raw material substance") in water; the raw material substance is added to the alkylamine aqueous solution in the reaction vessel with stirring; and the mixture is stirred for 1 to 2 hours. After stirring, water is added into the reaction vessel to form a mixed solution.

The mixed solution is preferably refluxed at a temperature of 60°C or higher and 80°C or lower for a period of 10 hours or more and 50 hours or less with stirring. In addition, if necessary, refluxing may be continued at a temperature of 100°C or higher and 120°C or lower for a period of 5 hours or more and 20 hours or less with stirring. The mixed solution is then cooled to room temperature, the residual alkylamine is quenched dropwise with concentrated hydrochloric acid, and the resulting reactant is suction-filtered for solid-liquid separation. The resulting solid reactant can be washed with water and dried to obtain N,N'-dialkylbicyclo[2.2.2]oct-7-ene-2,3;5,6-tetracarboxydiimide or N,N'-dialkylbicyclo[2.2.2]octane-2,3;5,6-tetracarboxydiimide (hereinafter also referred to as "imide compound").

Examples of the alkylamine include methylamine, ethylamine, n-propylamine, isopropylamine, n-butylamine, sec-butylamine, isobutylamine, and tert-butylamine.

### Second Step

### <Embodiment Satisfying Condition (1)>

The imide compound obtained in the first step and an organic solvent can be charged into a reaction vessel equipped with a reflux tube, and stirred at, for example, room temperature (20°C or higher and 30°C or lower) for a period of 10 minutes or more and 60 minutes or less to obtain a solution containing the imide compound. The organic solvent is not particularly limited, and examples thereof include THF (tetrahydrofuran), cyclopentyl methyl ether, methyl tertiary-butyl ether, and dioxane. Examples of the atmosphere for stirring include inert gas atmospheres such as a nitrogen atmosphere, an argon atmosphere, and a helium atmosphere.

A reducing agent described below and an organic solvent may be added to the imide compound obtained in the first step instead of obtaining a solution containing the imide compound and the organic solvent.

A solution obtained by mixing a reducing agent described below and an organic solvent can then be added to the solution containing the imide compound or to the imide compound, and the mixture is refluxed in a nitrogen atmosphere at a temperature of, for example, 50°C or higher and 80°C or lower for a period of 5 hours or more and 25 hours or less with stirring to obtain a reaction solution. The resulting reaction solution is then cooled to room temperature, and the reaction is terminated by adding water and an alkali solution. The organic solvent is not particularly limited and may be the same as those described above. Examples of the alkaline solution include a sodium hydroxide solution.

The solid in the reaction solution is then suction-filtered to obtain a liquid phase containing the reactant. A filtration aid (such as celite) may be added to the reaction solution prior to suction filtration. The resulting liquid phase containing the reactant can then be concentrated while evaporating low-boiling components such as organic solvents and water from the liquid phase, e.g. using an evaporator, to obtain a liquid pyrrolidine derivative 1.

Examples of the reducing agent satisfying the condition (1) include sodium bis(2-methoxyethoxy)aluminum hydride, lithium triethylborohydride, diisobutylaluminum hydride, diborane, lithium borohydride, and sodium borohydride. Among these, sodium bis(2-methoxyethoxy)aluminum hydride is particularly preferred. In the second step, by using a reducing agent such as sodium bis(2-methoxyethoxy)aluminum hydride, which has high solubility in organic solvents and exhibits stable reactivity without being affected by environmental atmospheres such as humidity and air, a pyrrolidine derivative 1 with highly dispersed hardly-water-soluble impurities can be easily obtained.

### <Embodiment Not Satisfying Condition (1)>

In the second step, a reducing agent described below and an organic solvent can be charged into a reaction vessel equipped with a reflux tube, and stirred at, for example, room temperature (20°C or higher and 30°C or lower) for a period of 10 minutes or more and 60 minutes or less to obtain a solution containing the reducing agent that does not satisfy the condition (1). Examples of the reducing agent that does not satisfy the condition (1) include lithium aluminum hydride, potassium borohydride, nickel borohydride, zinc borohydride, and potassium aluminum hydride. The organic solvent may be the same as those described above. Examples of the atmosphere for stirring include inert gas atmospheres such as a nitrogen atmosphere, an argon atmosphere, and a helium atmosphere.

A solution obtained by mixing the imide compound obtained in the first step and an organic solvent can then be added into the reaction vessel, and the mixture is refluxed in a nitrogen atmosphere at a temperature of, for example, 50°C or higher and 80°C or lower for a period of 5 hours or more and 25 hours or less with stirring to obtain a reaction solution. Subsequently, a pyrrolidine derivative 1 can be obtained in the same manner as in the case of satisfying the condition (1) in the second step.

In the case of hydrogen reduction as shown in the scheme (S3), for example, the N,N'-dialkylbicyclo[2.2.2]oct-7-ene-2,3;5,6-tetracarboxydiimide obtained in the first step and a palladium-activated carbon (Pd 10%) catalyst (manufactured by FUJIFILM Wako Pure Chemical Corp.) are charged into a sealed reaction vessel (50 mL) with a pressure gauge, and reduced with hydrogen by pressurizing with hydrogen (H₂) gas to 3 MPa or more and 10 MPa or less, and setting the temperature of the reaction vessel in a range of 100°C or higher and 200°C or lower, thereby obtaining a pyrrolidine derivative 1.

### Third Step

### <Embodiment Satisfying Condition (2)>

The pyrrolidine derivative 1 obtained in the second step can be reacted with an alkyl halide in the presence of an organic solvent in a molar ratio of 13 times or less relative to the pyrrolidine derivative 1 to obtain an N,N,N',N'-tetraalkylbicyclo[2.2.2]oct-7-ene-2,3;5,6-dipyrrolidinium salt or an N,N,N',N'-tetraalkylbicyclo[2.2.2]octane-2,3;5,6-dipyrrolidinium salt (hereinafter also referred to as "pyrrolidine derivative 2").

This reaction is a Menschutkin reaction in which a tertiary amine is reacted with a halogen compound to be quaternized to form a quaternary ammonium salt. The Menschutkin reaction is a nucleophilic substitution reaction, and all pyrrolidine derivatives 2 are tertiary amines substituted with alkyl groups. Therefore, while the electron density on the nitrogen atom is high and the basicity is high, the nucleophilicity is significantly reduced due to steric hindrance. The nitrogen atom acts as a base, and as a side reaction, the solvent ethanol and ethyl iodide easily react to form diethyl ether. Therefore, in order to improve both the yield by suppressing the side reaction and selectively obtaining a pyrrolidine derivative 2, and the productivity by shortening the reaction time, attention is paid to the amount of ethanol added as a solvent.

Specifically, as a result of scrutinizing by fine-tuning the amount of ethanol added at various levels, when the molar ratio of the organic solvent present in the reaction is reduced to 13 times or less relative to the pyrrolidine derivative 1, a pyrrolidine derivative 2 can be obtained in which the reaction time of the Menschutkin reaction is shortened by 80% or more, and the side reaction between the alkyl halide and the organic solvent is suppressed to improve the yield by 30% or more compared to conventional methods.

The use of alcohol as the organic solvent is preferable from the viewpoint of suppressing the formation of a dialkyl ether produced by a side reaction between the alcohol as the organic solvent and an alkyl halide. It is also preferable to directly add an alkyl halide to the pyrrolidine derivative 1 without using an organic solvent to obtain a pyrrolidine derivative 2 (such a method may be referred to as a "direct addition method").

Although the reaction time depends on the reaction volume and cannot be generalized, the reaction of the pyrrolidine derivative 1 with the alkyl halide reacts rapidly due to the small amount of the organic solvent, and thus the reaction is preferably completed within 30 minutes, more preferably within 20 minutes, and even more preferably within 15 minutes, to obtain a pyrrolidine derivative 2.

The alkyl halide added directly to the pyrrolidine derivative 1 is preferably added at a rate of 20 mL/min or less, more preferably 3 mL/min or more and 10 mL/min or less, in order to suppress temperature rise due to rapid reaction.

After completion of the reaction, for example, the resulting solid content may be washed with an organic solvent such as acetone, filtered with filter paper, and dried naturally to obtain a pyrrolidine derivative 2. When the content of hardly-water-soluble impurities is reduced, the yield of the target pyrrolidine derivative tends to be high.

### <Embodiment Not Satisfying Condition (2)>

The pyrrolidine derivative 1 obtained in the second step, which satisfies the condition (1), and, for example, an organic solvent in a molar ratio of more than 13 times relative to the pyrrolidine derivative 1 can be charged into a reaction vessel equipped with a reflux tube, and stirred at room temperature (20°C or higher and 30°C or lower) for a period of 10 minutes or more and 60 minutes or less to obtain a reaction solution. An alkyl halide solution is added to the reaction solution, the mixture is refluxed at a temperature of 50°C or higher and 80°C or lower for a period of 5 hours or more and 100 hours or less with stirring, the reaction solution is then cooled for 12 hours or more, the solid precipitated in the reaction solution is filtered, and the resulting solid is washed with an organic solvent such as acetone.

The precipitated solid is further refluxed in an organic solvent such as acetone, the solid is suction-filtered, and the resulting solid is further washed with an organic solvent such as acetone and dried to obtain a pyrrolidine derivative 2. In the present embodiment, the use of the pyrrolidine derivative 1 with suppressed formation of hardly-water-soluble impurities, which is obtained in the second step that satisfies the condition (1), enables obtaining a pyrrolidine derivative 2 in which the hardly-water-soluble impurities are highly dispersed and the formation of the hardly-water-soluble impurities is reduced in the production of MSE-type zeolites. The method of precipitating the pyrrolidine derivative 2 in the reaction solution described above may be referred to as "crystallization method".

Examples of the alkyl halide include methyl iodide, ethyl iodide, propyl iodide, butyl iodide, methyl bromide, ethyl bromide, propyl bromide, and butyl bromide.

The method for producing a pyrrolidine derivative preferably satisfies either the condition (1) in the second step or the condition (2) in the third step, from the viewpoint of obtaining N,N'-dialkylbicyclo[2.2.2]oct-7-ene-2,3;5,6-dipyrrolidine and/or N,N'-dialkylbicyclo[2.2.2]octane-2,3;5,6-dipyrrolidine that is a pyrrolidine derivative containing quaternary ammonium cations having a bicyclo framework with a peak intensity ratio (I/II) in a range of 0.4 or more and 1.5 or less.

The resulting pyrrolidine derivative can be analyzed by ¹H-NMR and ¹³C-NMR measurements to confirm the formation of N,N'-dialkylbicyclo[2.2.2]oct-7-ene-2,3;5,6-dipyrrolidine and/or N,N'-dialkylbicyclo[2.2.2]octane-2,3;5,6-dipyrrolidine.

### MSE-Type Zeolite

The MSE-type zeolite of the present invention preferably has an X-ray diffraction spectrum with a highest peak intensity A in a diffraction angle (2θ) range of 21.0° to 22.0° representing the (420) plane, and a highest peak intensity B in a diffraction angle (2θ) range of 22.6° to 23.6° representing the (115) plane, wherein a peak intensity ratio (A/B) of the peak intensity A to the peak intensity B is in a range of 1.25 or more and 2.50 or less. When the peak intensity ratio (A/B) falls within the range of 1.25 or more and 2.50 or less, the MSE-type zeolite contains a large amount of an MSE-type crystal phase and a low content of crystal phases other than the MSE-type crystal phase, which cause defects, and has excellent heat resistance. The MSE-type zeolite having a peak intensity ratio (A/B) in the range of 1.25 or more and 2.50 or less may be an MSE-type zeolite containing an organic structure directing agent (also referred to as "MSE-type zeolite (with OSDA)"), or may be an MSE-type zeolite containing no organic structure directing agent (also referred to as "MSE-type zeolite (without OSDA)").

The MSE-type zeolite is preferably synthesized by using the aforementioned pyrrolidine derivative containing quaternary ammonium cations having a bicyclo framework as an organic structure directing agent. In the MSE-type zeolite containing the aforementioned pyrrolidine derivative as an organic structure directing agent, the peak intensity ratio (A/B) is more preferably in a range of 1.30 or more and 2.50 or less. When the MSE-type zeolite (with OSDA) has a peak intensity ratio (A/B) in the range of 1.30 or more and 2.50 or less, the formation of crystal phases other than the MSE-type crystal phase is suppressed.

The MSE-type zeolite synthesized using the aforementioned pyrrolidine derivative as an organic structure directing agent can be calcined, for example, at a temperature of 600°C or higher and 800°C or lower in an air atmosphere after synthesis to remove the organic structure directing agent. The MSE-type zeolite (without OSDA) from which the organic structure directing agent is removed preferably has a peak intensity ratio (A/B) in a range of 1.25 or more and 2.50 or less, more preferably in a range of 1.26 or more and 2.00 or less, and even more preferably in a range of 1.28 or more and 1.80 or less. When the MSE-type zeolite (without OSDA) has a peak intensity ratio (A/B) in the range of 1.25 or more and 2.50 or less, the formation of crystal phases other than the MSE-type crystal phase is suppressed.

The MSE-type zeolite (with OSDA or without OSDA) preferably has a crystallite size, as determined from a peak having the peak intensity A, in a range of 20 nm or more and 80 nm or less. When the crystallite size of the MSE-type zeolite, as determined from a peak having the peak intensity A, falls within the range of 20 nm or more and 80 nm or less, the crystallite size of the MSE-type crystal phase is within a suitable range, the content of crystal phases other than the MSE-type crystal phase is low, and the MSE-type zeolite has high heat resistance. The crystallite size of the MSE-type zeolite, as determined from a peak having the peak intensity A, may be 25 nm or more, may be 30 nm or more, or may be 40 nm or more; and may be 75 nm or less.

The crystallite size of the MSE-type zeolite (with OSDA), as determined from a peak having the peak intensity A, is preferably in a range of 35 nm or more and 80 nm or less, may be 40 nm or more, or may be 50 nm or more; and may be 75 nm or less.

The crystallite size of the MSE-type zeolite (without OSDA), as determined from a peak having the peak intensity A, is preferably in a range of 20 nm or more and 80 nm or less, may be 30 nm or more, or may be 40 nm or more; and may be 75 nm or less, may be 70 nm or less, may be 65 nm or less, or may be 60 nm or less.

The MSE-type zeolite (with OSDA) preferably has a peak intensity ratio (A/α) of the peak intensity A to the peak intensity α in a range of 0.55 or more and 0.95 or less. When the peak intensity ratio (A/α) falls within the range of 0.55 or more and 0.95 or less, the MSE-type zeolite (with OSDA) contains a large amount of the MSE-type crystal phase and a low content of crystal phases other than the MSE-type crystal phase, which cause defects, and has excellent heat resistance. The peak intensity ratio (A/α) of the MSE-type zeolite (with OSDA) may be 0.60 or more, or 0.65 or more; and may be 0.90 or less.

The MSE-type zeolite (without OSDA) preferably has a peak intensity ratio (A/α) in a range of 0.28 or more and 0.60 or less. When the peak intensity ratio (A/α) falls within the range of 0.28 or more and 0.60 or less, the MSE-type zeolite (without OSDA) contains a low content of crystal phases other than the MSE-type crystal phase, which cause defects, and has excellent heat resistance. The peak intensity ratio (A/α) of the MSE-type zeolite (without OSDA) may be 0.30 or more, or 0.35 or more; and may be 0.58 or less, or may be 0.55 or less.

The MSE-type zeolite (without OSDA) preferably has an X-ray diffraction spectrum with a peak intensity ratio (C/α) of a highest peak intensity C in a diffraction angle (2θ) range of 9.2° to 10.2° representing the (200) plane to the peak intensity α in a range of 0.15 or more and 0.50 or less. When the peak intensity ratio (C/α) falls within the range of 0.15 or more and 0.50 or less, the MSE-type zeolite contains a large amount of the MSE-type crystal phase and a low content of crystal phases other than the MSE-type crystal phase, which cause defects, and has excellent heat resistance. In the X-ray diffraction spectrum of the MSE-type zeolite, the peak in the diffraction angle (2θ) range of 9.2° to 10.2° representing the (200) plane appears as a peak having a large peak intensity in the X-ray diffraction spectrum of the MSE-type zeolite (without OSDA) from which the organic structure directing agent is removed. The peak intensity ratio (C/α) of the MSE-type zeolite (without OSDA) may be 0.20 or more, or 0.25 or more; and may be 0.45 or less, or may be 0.40 or less.

The MSE-type zeolite (with OSDA or without OSDA) preferably has a SiO₂/Al₂O₃ molar ratio of 10 or more and 600 or less. The SiO₂/Al₂O₃ molar ratio of the MSE-type zeolite may be 15 or more, may be 18 or more, or may be 20 or more; and may be 500 or less, may be 300 or less, or may be 100 or less. When the SiO₂/Al₂O₃ molar ratio of the MSE-type zeolite is 10 or more, the MSE-type zeolite has a stable crystal structure and excellent heat resistance. When the SiO₂/Al₂O₃ molar ratio of the MSE-type zeolite is 600 or less, metal ions are more easily ion-exchanged with a group containing oxygen atoms serving as a BrOnsted acid site adjacent to Al atoms in the multipore zeolite when the MSE-type zeolite is used as a hydrocarbon adsorbent. The SiO₂/Al₂O₃ molar ratio contained in the MSE-type zeolite can be determined by measuring the amount of Si and the amount of Al by elemental analysis using, for example, a scanning fluorescent X-ray analyzer (ZSX Primus II, manufactured by Rigaku Corp.), and calculating the SiO₂/Al₂O₃ molar ratio from the measured amounts of Si and Al.

As described above, the MSE-type zeolite of the present invention has a low content of crystal phases other than the MSE-type crystal phase, which cause defects. Therefore, the accessible volume, which indicates the adsorption capacity of the zeolite, is high, resulting in an MSE-type zeolite with excellent adsorption capacity.

### Method for Producing MSE-Type Zeolite

The method for producing an MSE-type zeolite includes a step of preparing a raw material mixture containing a silica source, an aluminum source, an alkali metal source, an organic structure directing agent (OSDA), and water, and a step of heating and crystallizing the raw material mixture to obtain an MSE-type zeolite.

The silica source can use, for example, a silicon-containing compound. Specific examples thereof include wet-process silica, dry-process silica, colloidal silica, sodium silicate, potassium silicate, and aluminosilicate gel. These silica sources can be used alone or in combination of two or more types thereof. Among these silica sources, silica (silicon dioxide), aluminosilicate gel, and colloidal silica are preferably used because they are less likely to generate by-products and can produce the desired MSE-type zeolite.

The aluminum source can use, for example, a water-soluble aluminum-containing compound. Specific examples thereof include sodium aluminate, aluminum nitrate, aluminum sulfate, aluminum hydroxide, and aluminosilicate gel. These aluminum sources can be used alone or in combination of two or more types thereof. Among these aluminum sources, sodium aluminate and aluminosilicate gel are preferably used since they are less likely to generate by-products and can produce the desired MSE-type zeolite.

The alkali metal source can use, for example, cesium hydroxide, potassium hydroxide, and sodium hydroxide. When sodium silicate is used as the silica source or sodium aluminate is used as the aluminum source, sodium, which is an alkali metal component contained therein, is also an alkali metal component. The alkali metal source is calculated as the sum of all alkali metal components in the reaction mixture.

The organic structure directing agent can use the aforementioned pyrrolidine derivative containing quaternary ammonium cations. Specific examples thereof include pyrrolidine derivatives containing N,N,N',N'-tetraalkylbicyclo[2.2.2]oct-7-ene-2,3:5,6-dipyrrolidinium cation and/or N,N,N',N'-tetraalkylbicyclo[2.2.2]octane-2,3:5,6-dipyrrolidinium cation.

The order of addition of each raw material in preparing the raw material mixture can be in a manner that facilitates obtaining a uniformly mixed raw material mixture. For example, at room temperature, a uniformly mixed raw material mixture can be obtained by adding and dissolving an aluminum source to an alkali metal source acting as a mineralizing agent, adding a silica source thereto, and then stirring and mixing the materials. The raw material mixture can be crystallized by heating at a temperature of 70°C or higher and 240°C or lower. After heating, the crystallized powder can be separated from the mother liquor by filtration, washed with water or deionized water, and dried to obtain an MSE-type zeolite. The MSE-type zeolite can be specifically produced by referring to the method described in PCT/JP2020/029445, except that the aforementioned pyrrolidine derivative containing quaternary ammonium cations is used as the organic structure directing agent.

Whether or not the resulting zeolite is an MSE-type zeolite can be confirmed by measurement using a powder X-ray diffractometer. In the X-ray diffraction spectrum obtained using a powder X-ray diffractometer, if there is a diffraction peak at a diffraction angle (2θ) position of 6.56° ± 0.15°, 6.8° ± 0.15°, 8.04° ± 0.15°, 19.38° ± 0.15°, 19.42° ± 0.15°, 21.68° ± 0.15°, 22.28° ± 0.15°, 22.32° ± 0.15°, 22.84° ± 0.15°, or 27.5° ± 0.15°, it can be confirmed that it is an MSE-type zeolite.

### EXAMPLES

Hereinafter, the present invention will be further described in detail based on Examples and Comparative Examples. The present invention is not limited to the following Examples.

In the scheme (ES1), the scheme (ES2), and the scheme (ES3) shown in Examples described below, the "Et" represents an ethyl group, the reducing agent "Red-Al" represents sodium bis(2-methoxyethoxy)aluminum hydride, the reducing agent "LiAlH₄" represents lithium aluminum hydride, and other notations have the same meanings as those in the scheme (S1), the scheme (S2), and the scheme (S3).

### Example 1 - Pyrrolidine Derivative

According to the following scheme (ES1), N,N,N',N'-tetraethylbicyclo[2.2.2]oct-7-ene-2,3;5,6-dipyrrolidinium iodide (d) was produced.

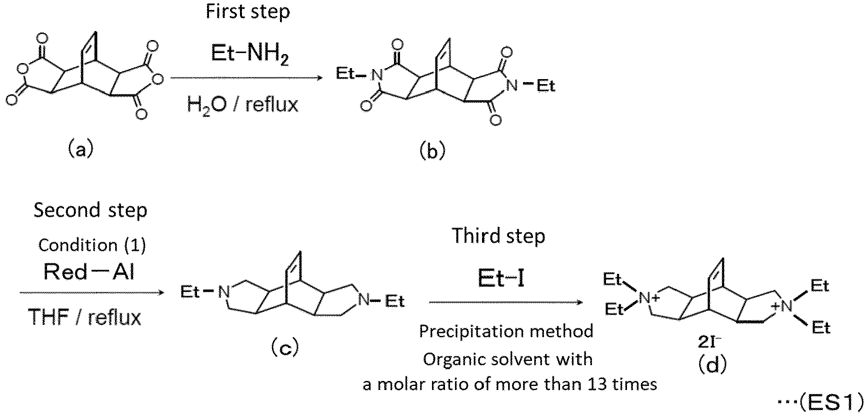

### Example 1 - Pyrrolidine Derivative - First Step

A reaction vessel was prepared with a reflux condenser and a thermometer attached to a 1,000 mL three-necked round bottom flask equipped with a magnetic stirrer. The reaction vessel was then charged with a 70% by mass ethylamine aqueous solution (30.2 mL (24.16 g)), followed by the addition of exo, exo-bicyclo[2.2.2]oct-7-ene-2,3;5,6-tetracarboxylic dianhydride (a) (10.0 g) in several portions with vigorous stirring. The mixture was stirred at room temperature (approximately 25°C) for 2 hours, and then water (65.2 mL) was added to obtain an aqueous solution 1. The aqueous solution 1 in the reaction vessel was refluxed with stirring at 70°C for 24 hours and then refluxed with stirring at 100°C for 18 hours to obtain a reactant 1. The resulting reactant 1 was cooled to room temperature (approximately 25°C), and the residual alkylamine was adjusted to pH 2 using concentrated hydrochloric acid in a dropwise manner. The resulting solid reactant 1 was suction-filtered, washed with water (230 mL), and dried on a desiccant in a vacuum desiccator to obtain 11.20 g (92% yield) of N,N'-diethylbicyclo[2.2.2]oct-7-ene-2,3;5,6-tetracarboxydiimide (b).

### Example 1 - Pyrrolidine Derivative - Second Step

The atmosphere in a 1,000 mL three-necked round bottom flask equipped with a magnetic stirrer, a thermometer, and a pressure-equalizing dropping funnel sealed with a septum cap was totally replaced with nitrogen. A reflux condenser was attached to the three-necked round bottom flask to prepare a reaction vessel. The N,N'-dialkylbicyclo[2.2.2]oct-7-ene-2,3;5,6-tetracarboxydiimide (b) (10.70 g) obtained in the first step and dried THF (tetrahydrofuran) (192.6 mL) were charged into the reaction vessel and stirred at 25°C for 0.5 hour in a nitrogen atmosphere (100% N₂ gas flowing into the reaction vessel at 30 mL/min). Then, a toluene solution (60.78 g) containing 70% by mass of sodium bis(2-methoxyethoxy)aluminum hydride (Red-Al) as a reducing agent that satisfies the condition (1) was prepared, the toluene solution containing Red-Al was mixed with dried THF (96.3 mL), and the mixed solution was dropped into the reaction vessel through the pressure-equalizing dropping funnel to obtain a reaction solution 2. The reaction solution 2 in the reaction vessel was refluxed at 67°C for 23 hours in a nitrogen atmosphere (100% N₂ gas flowing into the reaction vessel at 30 mL/min). The reaction solution 2 was then cooled to room temperature (25°C). Water (4.08 g) was added to the reaction solution 2, followed by the addition of sodium hydroxide (NaOH, 15% by mass concentration, 4.71 g), then water (12.24 g) to complete the reaction. The reaction solution 2 was then added with celite (2.0 g) as a filtration aid, and stirred at 25°C for 0.5 hour. After stirring, the reaction solution 2 was suction-filtered while being washed with THF to obtain a liquid phase containing a reactant 2. Then, using an evaporator, low-boiling components such as THF and water were distilled under reduced pressure at 60°C for 2 hours from the resulting liquid phase containing a reactant 2 to obtain N,N'-diethylbicyclo[2.2.2]oct-7-ene-2,3;5,6-dipyrrolidine (c). In Example 1, a pyrrolidine derivative 1 was produced using a reducing agent satisfying the condition (1) in the second step.

### Example 1 - Pyrrolidine Derivative - Third Step

A reaction vessel was prepared with a reflux condenser and a thermometer attached to a 1,000 mL three-necked round bottom flask equipped with a magnetic stirrer. The N,N'-diethylbicyclo[2.2.2]oct-7-ene-2,3;5,6-pyrrolidine (8.44 g) obtained in the second step and ethanol (75 mL) were then charged into the reaction vessel and stirred at 25°C for 0.5 hour to obtain a mixture 3. Ethyl iodide (11.0 mL, 137 mmol) and ethanol (5 to 10 mL) were mixed and added to the mixture 3, and the mixture was primary-refluxed at 60°C for 67 hours under dark room conditions to obtain a reaction solution 3. The organic solvent (ethanol) in the reaction solution 3 had a molar ratio of approximately 38 times relative to the N,N'-diethylbicyclo[2.2.2]oct-7-ene-2,3;5,6-pyrrolidine. The reaction solution 3 was then cooled, and the solid precipitated in the reaction solution 3 was suction-filtered and washed with acetone (60 mL). The resulting solid was added with acetone (50 mL) and secondary-refluxed at 0°C for 0.5 hours, and the solid was suction-filtered, washed with acetone (50 mL), and dried in vacuum at 40°C for 3 hours to obtain N,N,N',N'-tetraethylbicyclo[2.2.2]oct-7-ene-2,3;5,6-dipyrrolidinium iodide (d) (65.3% yield). In the third step of Example 1, the organic solvent had a molar ratio of approximately 40 times relative to the N,N'-diethylbicyclo[2.2.2]oct-7-ene-2,3;5,6-pyrrolidine. In the third step, a pyrrolidine derivative 2 was produced by a crystallization method not satisfying the condition (2).

### Example 2 - Pyrrolidine Derivative

According to the following scheme (ES2), N,N,N',N'-tetraethylbicyclo[2.2.2]oct-7-ene-2,3;5,6-dipyrrolidinium iodide (d) was produced.

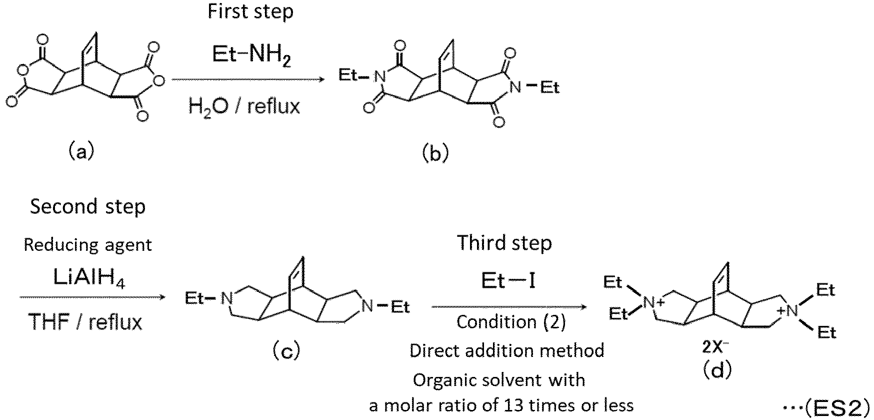

### Example 2 - Pyrrolidine Derivative - First Step

N,N'-diethylbicyclo[2.2.2]oct-7-ene-2,3;5,6-tetracarboxydiimide (b) was obtained in the same manner as in the first step of Example 1.

### Example 2 - Pyrrolidine Derivative - Second Step

Lithium aluminum hydride (LiAlH₄, 4.03 g) as a reducing agent not satisfying the condition (1) and dried THF (260 mL) were mixed and charged into a reaction vessel, and stirred at 25°C for 0.5 hour. The N,N'-dialkylbicyclo[2.2.2]oct-7-ene-2,3;5,6-tetracarboxydiimide (10.70 g) obtained in the first step and dried THF (92 mL) were then mixed, and the mixed solution was dropped into the reaction vessel through the funnel to obtain a reaction solution 2. The reaction solution 2 in the reaction vessel was refluxed at 67°C for 23 hours in an inert gas atmosphere (100% N₂ gas flowing into the reaction vessel at 30 mL/min). The reaction solution 2 was then cooled to room temperature (25°C). Water (4.08 g) was added to the reaction solution 2, followed by the addition of sodium hydroxide (NaOH, 15% by mass concentration, 4.71 g), then water (12.24 g) to complete the reaction. The reaction solution 2 was then filtered while being washed with THF to obtain a liquid phase containing a reactant 2. Then, using an evaporator, low-boiling components such as THF and water were distilled under reduced pressure at 60°C for 2 hours from the resulting liquid phase containing a reactant 2 to obtain N,N'-diethylbicyclo[2.2.2]oct-7-ene-2,3;5,6-dipyrrolidine (c). In Example 2, a pyrrolidine derivative 1 was produced using a reducing agent not satisfying the condition (1) in the second step.

### Example 2 - Pyrrolidine Derivative - Third Step

The N,N'-diethylbicyclo[2.2.2]oct-7-ene-2,3;5,6-dipyrrolidine (c) (10.0 g) obtained in the second step was placed in an open vessel, and ethyl iodide (13.03 mL, 25.4 g) was added directly to the vessel and mixed to obtain a reaction solution 3. After the addition of ethyl iodide, the reaction solution 3 became cloudy in 3 to 5 minutes, and within 10 minutes, the entire solution solidified while generating heat to 110°C. The resulting solid was washed with acetone, filtered, and dried to obtain N,N,N',N'-tetraethylbicyclo[2.2.2]oct-7-ene-2,3;5,6-dipyrrolidinium iodide (d) (84.1% yield). Although no organic solvent was added to N,N'-diethylbicyclo[2.2.2]oct-7-ene-2,3;5,6-pyrrolidine in the third step of Example 2, the same yield and synthesis time were obtained even when ethanol as an organic solvent was added up to approximately 12 times the molar ratio relative to the pyrrolidine. In Example 2, a pyrrolidine derivative 2 was produced by a direct addition method satisfying the condition (2) in the third step.

### Example 3 - Pyrrolidine Derivative

According to the following scheme (ES3), N,N,N',N'-tetraethylbicyclo[2.2.2]oct-7-ene-2,3;5,6-dipyrrolidinium iodide (d) was produced.

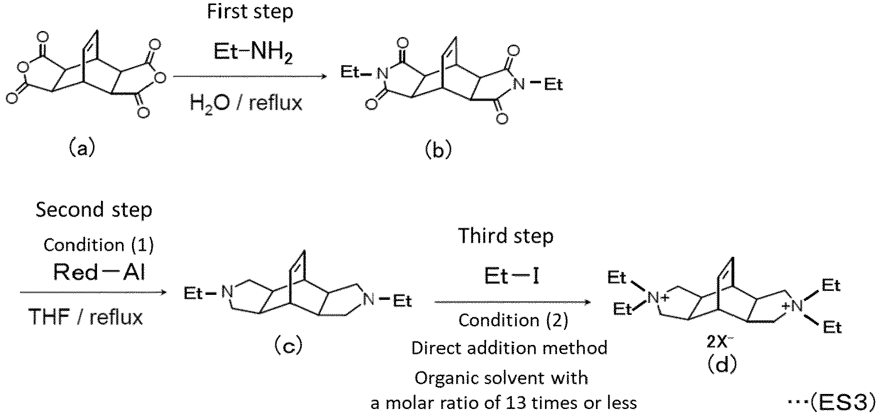

### Example 3 - Pyrrolidine Derivative - First Step

N,N'-diethylbicyclo[2.2.2]oct-7-ene-2,3;5,6-tetracarboxydiimide (b) was obtained in the same manner as in the first step of Example 1.

### Example 3 - Pyrrolidine Derivative - Second Step

N,N'-diethylbicyclo[2.2.2]oct-7-ene-2,3;5,6-dipyrrolidine (c) was obtained in the same manner as in the second step of Example 1. In Example 3, a pyrrolidine derivative 1 was produced using a reducing agent satisfying the condition (1) in the second step.

### Example 3 - Pyrrolidine Derivative - Third Step

N,N,N',N'-tetraethylbicyclo[2.2.2]oct-7-ene-2,3;5,6-dipyrrolidinium iodide (d) was obtained in the same manner as in the third step of Example 2. Although no organic solvent was added to N,N'-diethylbicyclo[2.2.2]oct-7-ene-2,3;5,6-pyrrolidine in the third step of Example 3, the same yield and reaction time were obtained even when an organic solvent was added up to approximately 12 times the molar ratio relative to the pyrrolidine. In Example 3, a pyrrolidine derivative 2 was produced by a direct addition method satisfying the condition (2) in the third step.

### Comparative Example 1 - Pyrrolidine Derivative

According to the following scheme (ES4), N,N,N',N'-tetraethylbicyclo[2.2.2]oct-7-ene-2,3;5,6-dipyrrolidinium iodide (d) was produced.

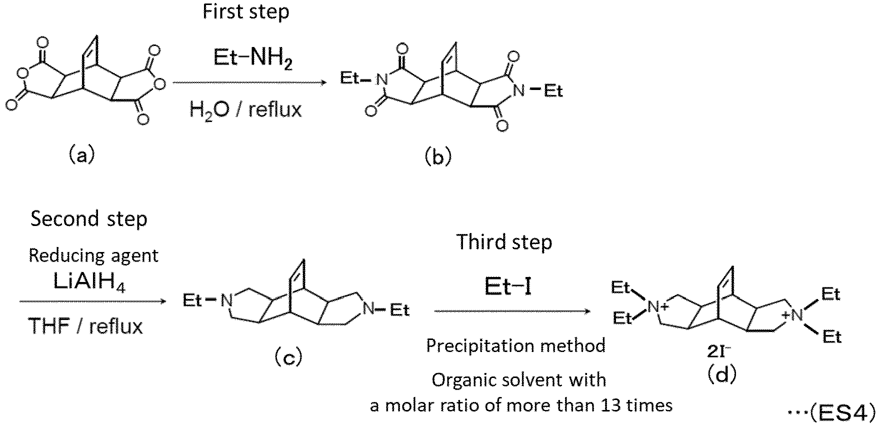

### Comparative Example 1 - Pyrrolidine Derivative - First Step

N,N'-diethylbicyclo[2.2.2]oct-7-ene-2,3;5,6-tetracarboxydiimide (b) was obtained in the same manner as in the first step of Example 1.

### Comparative Example 1 - Pyrrolidine Derivative - Second Step

N,N'-diethylbicyclo[2.2.2]oct-7-ene-2,3;5,6-dipyrrolidine (c) (pyrrolidine derivative 1) was obtained using a reducing agent not satisfying the condition (1) in the same manner as in the second step of Example 2.

### Comparative Example 1 - Pyrrolidine Derivative - Third Step

N,N,N',N'-tetraethylbicyclo[2.2.2]oct-7-ene-2,3;5,6-dipyrrolidinium iodide (d) (pyrrolidine derivative 2) was obtained by a crystallization method not satisfying the condition (2) in the same manner as in the third step of Example 1. In the third step of Comparative Example 1, the molar ratio of the organic solvent to N,N'-diethylbicyclo[2.2.2]oct-7-ene-2,3;5,6-pyrrolidine was approximately 40 times.

### X-Ray Diffraction Spectrum

For each pyrrolidine derivative 2 in Examples and Comparative Examples, an X-ray diffraction spectrum was measured using an X-ray diffractometer (XRD, model number: RINT-TTR III, manufactured by Rigaku Corp.) under the following measurement conditions. The highest peak intensity I and the diffraction angle (2θ) indicating the peak intensity I in the diffraction angle (2θ) range of 14.0° to 16.0° were measured from the X-ray diffraction spectrum measured for each pyrrolidine derivative 2 in Examples and Comparative Examples. In addition, the highest peak intensity II and the diffraction angle (2θ) indicating the peak intensity II in the diffraction angle (2θ) range of 17.0° to 19.0° were measured from the X-ray diffraction spectrum of each pyrrolidine derivative 2, to determine a peak intensity ratio (I/II). In addition, the peak intensity α representing the (116) plane of α-aluminum oxide, which is the standard material 674a distributed by the National Institute of Standards and Technology, was measured under the same conditions as those for measuring each pyrrolidine derivative 2 in Examples and Comparative Examples, to determine a peak intensity ratio (I/α) and a peak intensity ratio (II/α).

### Measurement Conditions

Radiation source: CuKα (line focus), wavelength: 1.5418 Å
Operation axis: 2θ/θ, measurement method: continuous, counting unit: cps
Initiation angle: 5°, termination angle: 80°, number of integration times: 1 time
Sampling width: 0.02°, scanning speed: 20°/min
Voltage: 50 kV, Current: 300 mA
Divergence slit: 2/3°, divergence vertical restriction slit: 10 mm
Scattering slit: opening, light-receiving slit: opening
Attachment: ASC-48
Slit: slit for D/teX Ultra
Detector: D/teX Ultra

### Crystallite Size

A crystallite size was determined from a peak having the peak intensity I according to Scherrer's formula in the X-ray diffraction spectrum measured for each pyrrolidine derivative 2 in Examples and Comparative Examples, specifically, it was determined by measuring a half value width (full width at half maximum) of a peak having the peak intensity I in the measured X-ray diffraction spectrum using an integrated powder X-ray analysis software (software name: PDXL2, manufactured by Rigaku Corp.) and calculating according to the following Scherrer's formula.
Scherrer's formula: D = Kλ/β cos θ
D: Crystallite size, K: Scherrer constant (1.333), λ: X-ray wavelength, β: Half value width (rad), θ: Diffraction angle

### BET Specific Surface Area

For each pyrrolidine derivative 2 in Example 2 and Comparative Example 1, a BET specific surface area was determined by a BET one-point method using a BET specific surface area measuring apparatus (model number: BELSORP MR6, manufactured by MicrotrackBEL Corp.).

### SEM Micrograph

For each pyrrolidine derivative 2 in Example 2 and Comparative Example 1, an SEM micrograph was obtained using an ultra-high resolution Schottky scanning electron microscope (model number: SU7000, manufactured by Hitachi High-Tech Corp.). A primary particle diameter of each pyrrolidine derivative 2 was determined from each SEM micrograph.

The production conditions, the peak intensity at each diffraction angle (2θ) obtained from the X-ray diffraction spectrum, the peak intensity ratios, and the crystallite size of each pyrrolidine derivative 2 in Examples and Comparative Examples are shown in Table 1.

**[Table 1]**

| | Production conditions | | Peak intensity I | | Peak intensity II | | Peak intensity ratio (I / II) | Peak intensity ratio (I / α) | Peak intensity ratio ( II/ α ) | Crystallite size (nm) |
|---|---|---|---|---|---|---|---|---|---|---|
| | | | 2θ = 14.0° - 16.0° | | 2θ = 17.0° - 199.0° | | | | | |
| | Second step | Third step | 2θ (deg) | Intensity I (cps) | 2θ (deg) | Intensity II (cps) | | | | |
| Example 1 | Condition (1) Red-Al | Crystallization method | 15.01 | 22900.0 | 18.08 | 29550.0 | 0.77 | 0.36 | 0.46 | 59.6 |
| Example 2 | LiAlH₄ | Condition (2) Direct addition method | 14.94 | 10650.0 | 18.00 | 22183.3 | 0.48 | 0.17 | 0.35 | 48.9 |
| Example 3 | Condition (1) Red-Al | Condition (2) Direct addition method | 15.04 | 13416.7 | 18.04 | 20433.3 | 0.66 | 0.21 | 0.32 | 108.6 |
| Comparative Example 1 | LiAlH₄ | Crystallization method | 14.87 | 62466.7 | 17.88 | 36850.0 | 1.70 | 0.98 | 0.58 | 123.1 |

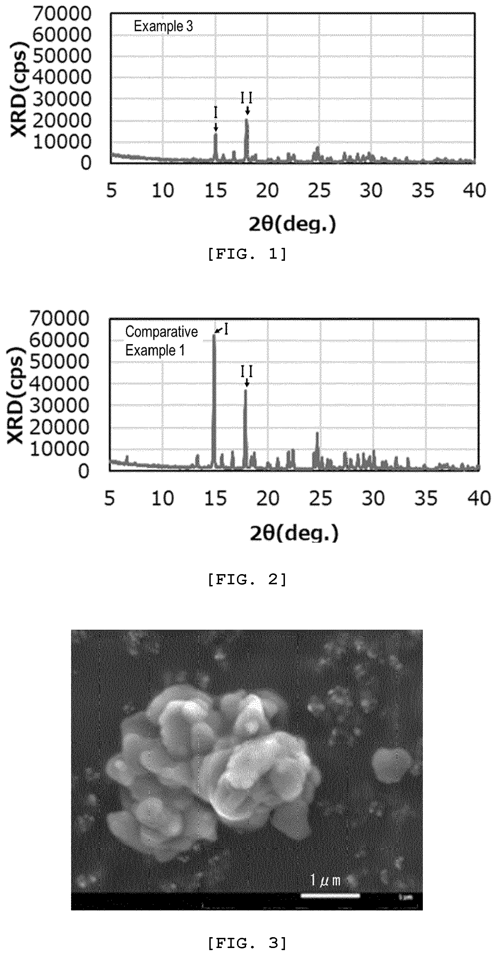

FIGS. 1 and 2 show the X-ray diffraction spectrum of the pyrrolidine derivative 2 in Example 3 and Comparative Example 1, respectively. The peak intensity II of the pyrrolidine derivative 2 in Example 3 shown in FIG. 1 was higher compared to the peak intensity I, whereas the peak intensity II of the pyrrolidine derivative 2 in Comparative Example 1 shown in FIG. 2 was lower compared to the peak intensity I.

FIG. 3 shows an SEM micrograph of the pyrrolidine derivative 2 in Example 2. The primary particle diameter of the pyrrolidine derivative 2 in Example 2 measured from the SEM micrograph was 0.3 to 1.2 µm, which was 3.0 µm or less. The BET specific surface area of the pyrrolidine derivative 2 in Example 2 was 4.0 m²/g. When the pyrrolidine derivative 2 in Example 2 was dissolved in water, no particulate material was visually observed. In other words, all of the pyrrolidine derivative 2 in Example 2 was dissolved in water, and no substances were observed that would be hardly-water-soluble impurities.

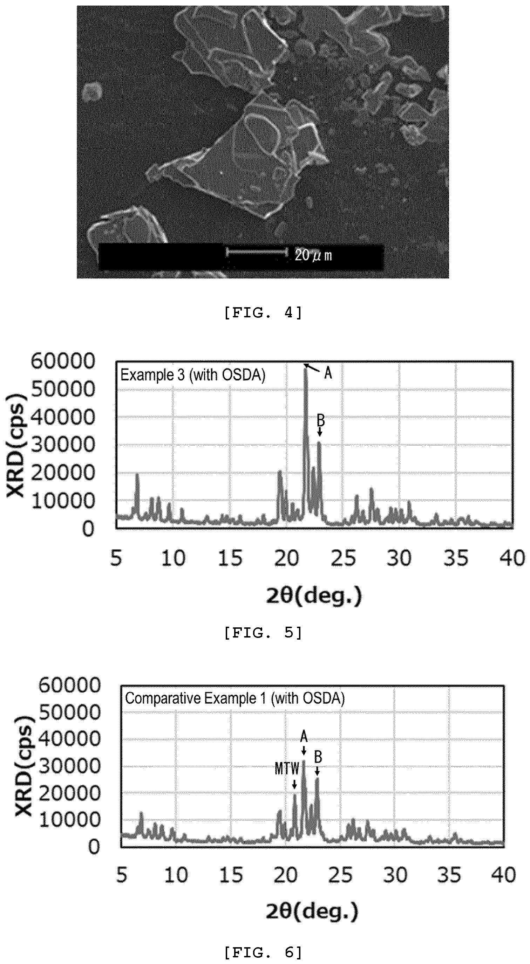

FIG. 4 shows an SEM micrograph of the pyrrolidine derivative 2 in Comparative Example 1. The primary particle diameter of the pyrrolidine derivative 2 in Comparative Example 1 measured from the SEM micrograph was 20 to 40 µm, which was more than 10.0 µm. The BET specific surface area of the pyrrolidine derivative 2 in Comparative Example 1 was 0.5 m²/g. When the pyrrolidine derivative 2 in Comparative Example 1 was dissolved in water, visually visible hardly-water-soluble black grains were observed in a ratio of approximately 10% by mass to 100% by mass of the pyrrolidine derivative 2. When this was measured by NMR, peaks other than those of the pyrrolidine derivative 2 were confirmed. Based on the particle diameter and visually observable color of the pyrrolidine derivative 2 in Comparative Example 1, it was presumed that the content of hardly-water-soluble impurities was high.

The ¹H-NMR and the ¹³C-NMR of each pyrrolidine derivative 2 in Example 2 and Comparative Example 1 were measured, and the results were compared with the values of N,N,N',N'-tetraethylbicyclo[2.2.2]oct-7-ene-2,3;5,6-dipyrrolidinium iodide (hereinafter also referred to as "literature values") disclosed in "Rapid Synthesis of an Aluminum-Rich MSE-Type Zeolite by the Hydrothermal Conversion of an FAU-Type Zeolite", S.Inagaki, et al., Chemistry A European Journal, Volume 19, Pages 7780-7786, June 10, 2013.

### ¹H-NMR Measurement Conditions

Apparatus: AVANCE NEO 600 manufactured by Bruker Corp.
Solvent: 400 MHz, CDCl₃, TMS, D₂O
¹³C-NMR Measurement Conditions
Apparatus: AVANCE NEO 600 manufactured by Bruker Corp.
Solvent: 100 MHz, CDCl₃, TMS, D₂O

The ¹H chemical shift (ppm) of the pyrrolidine derivative 2 in Example 2 and literature values are shown in Table 2, and the ¹³C chemical shift (ppm) of the pyrrolidine derivative 2 in Example 2 and literature values are shown in Table 3.

**[Table 2]**

| | ¹H chemical shift (ppm) | | | | | | | | | | | |
|---|---|---|---|---|---|---|---|---|---|---|---|---|
| Example 2 | | 0.74 | 1.28 | 1.66 | 1.81 | 2.13 | 2.83 | 2.90 | 3.30 | 3.80 | 4.77 | 6.44 |
| Literature values | | impurities | 1.30 | impurities | impurities | impurities | 2.87 | 2.93 | 3.33 | 3.83 | H₂O | 6.47 |

**[Table 3]**

| | ¹³C chemical shift (ppm) | | | | | | | | | | |
|---|---|---|---|---|---|---|---|---|---|---|---|
| Example 2 | | 7.23 | 8.10 | 20.06 | 32.94 | 39.86 | 52.56 | 55.52 | 64.30 | 134.07 | 185.79 |
| Literature values | | 7.27 | 8.12 | impurities | 32.87 | 39.75 | 52.50 | 55.43 | 64.25 | 134.05 | impurities |

The ¹H chemical shift (ppm) of the pyrrolidine derivative 2 in Comparative Example 1 and literature values are shown in Table 4, and the ¹³C chemical shift (ppm) of the pyrrolidine derivative 2 in Comparative Example 1 and literature values are shown in Table 5.

**[Table 4]**

| | ¹H chemical shift (ppm) | | | | | | | |
|---|---|---|---|---|---|---|---|---|
| Comparative Example 1 | | 1.17 | 2.75 | 2.80 | 3.20 | 3.70 | 4.70 | 6.34 |
| Literature values | | 1.30 | 2.87 | 2.93 | 3.33 | 3.83 | H₂O | 6.47 |

| | | | | | | | | |
|---|---|---|---|---|---|---|---|---|
| ** There is an error of approximately 0.13 ppm because chemical shift correction by TMS is not performed. | | | | | | | | |

**[Table 5]**

| | ¹³C chemical shift (ppm) | | | | | | | | |
|---|---|---|---|---|---|---|---|---|---|
| Comparative Example 1 | | 7.19 | 8.07 | 32.90 | 39.82 | 52.48 | 55.45 | 64.27 | 134.04 |
| Literature values | | 7.27 | 8.12 | 32.87 | 39.75 | 52.50 | 55.43 | 64.25 | 134.05 |

As shown in Tables 2 to 5, the pyrrolidine derivative 2 in Example 2 and the pyrrolidine derivative 2 in Comparative Example 1 each had the ¹H chemical shift by ¹H-NMR and the ¹³C chemical shift by ¹³C-NMR approximating the literature values listed above, thereby confirming that N,N,N',N'-tetraethylbicyclo[2.2.2]oct-7-ene-2,3;5,6-dipyrrolidinium iodide was produced.

### Example 1 - MSE-Type Zeolite

Deionized water (pure water), potassium hydroxide, aluminum hydroxide, the N,N,N',N'-tetraalkylbicyclo[2.2.2]oct-7-ene-2,3:5,6-dipyrrolidinium iodide in Example 1 as the organic structure directing agent, and colloidal silica (LUDOX (registered trademark) HS-40, manufactured by Sigma-Aldrich Japan) were prepared, and these were mixed to obtain a raw material composition having a composition with the following molar ratios of the raw materials.
SiO₂/Al₂O₃ = 10.0
KOH/Si = 0.375
N,N,N',N'-tetraalkylbicyclo[2.2.2]oct-7-ene-2,3:5,6-dipyrrolidinium cation/Si = 0.10
H₂O/Si = 30

The resulting raw material composition was charged into a sealed vessel and crystallized by heating at 160°C for 11 days in a static state. The crystallized raw material composition was subjected to solid-liquid separation, and washed with deionized water to recover crystals. The resulting crystals were dried at 100°C for 8 hours to obtain an MSE-type zeolite (with OSDA).

The resulting MSE-type zeolite (with OSDA) was heat-treated at 650°C for 3 hours in an air atmosphere to obtain an MSE-type zeolite from which the organic structure directing agent was removed (without OSDA).

The SiO₂/Al₂O₃ molar ratio of the resulting MSE-type zeolite was 10.

### Example 2 - MSE-Type Zeolite

An MSE-type zeolite (with OSDA) and an MSE-type zeolite from which the organic structure directing agent was removed (without OSDA) were obtained in the same manner as in Example 1, except that the N,N,N',N'-tetraalkylbicyclo[2.2.2]oct-7-ene-2,3:5,6-dipyrrolidinium iodide in Example 2 was used as the organic structure directing agent.

### Example 3 - MSE-Type Zeolite

An MSE-type zeolite (with OSDA) and an MSE-type zeolite from which the organic structure directing agent was removed (without OSDA) were obtained in the same manner as in Example 1, except that the N,N,N',N'-tetraalkylbicyclo[2.2.2]oct-7-ene-2,3:5,6-dipyrrolidinium iodide in Example 3 was used as the organic structure directing agent.

### Comparative Example 1 - MSE-Type Zeolite

An MSE-type zeolite (with OSDA) and an MSE-type zeolite from which the organic structure directing agent was removed (without OSDA) were obtained in the same manner as in Example 1, except that the N,N,N',N'-tetraalkylbicyclo[2.2.2]oct-7-ene-2,3:5,6-dipyrrolidinium iodide in Comparative Example 1 was used as the organic structure directing agent.

### X-Ray Diffraction Spectrum

For each MSE-type zeolite (with OSDA) and for each MSE-type zeolite from which the organic structure directing agent was removed (without OSDA) in Examples and Comparative Examples, an X-ray diffraction spectrum was measured using an X-ray diffractometer (XRD, model number: RINT-TTR III, manufactured by Rigaku Corp.) under the same conditions as those for measuring each pyrrolidine derivative. The highest peak intensity A and the diffraction angle (2θ) indicating the peak intensity A in the diffraction angle (2θ) range of 21.0° to 22.0° representing the (420) plane were measured from the X-ray diffraction spectrum measured for each MSE-type zeolite in Examples and Comparative Examples. In addition, the highest peak intensity B and the diffraction angle (2θ) indicating the peak intensity B in the diffraction angle (2θ) range of 22.6° to 23.6° representing the (115) plane were measured from the X-ray diffraction spectrum of each MSE-type zeolite, to determine a peak intensity ratio (A/B). In addition, the peak intensity α representing the (116) plane of α-aluminum oxide, which is the standard material 674a distributed by the National Institute of Standards and Technology, was measured under the same conditions as those for measuring each MSE-type zeolite in Examples and Comparative Examples, to determine a peak intensity ratio (A/α) and a peak intensity ratio (B/α). In addition, the highest peak intensity C and the diffraction angle (2θ) indicating the peak intensity B in the diffraction angle (2θ) range of 9.2° to 10.2° representing the (200) plane were measured from the X-ray diffraction spectrum of each MSE-type zeolite (without OSDA), to determine a peak intensity ratio (C/α).

### Crystallite Size

A crystallite size was determined from a peak having the peak intensity A according to the aforementioned Scherrer's formula in the X-ray diffraction spectrum measured for each MSE-type zeolite in Examples and Comparative Examples, specifically, it was determined by measuring a half value width (full width at half maximum) of a peak having the peak intensity A in the X-ray diffraction spectrum using an integrated powder X-ray analysis software (software name: PDXL2, manufactured by Rigaku Corp.) and calculating according to the Scherrer's formula.

The peak intensity at each diffraction angle (2θ) obtained from the X-ray diffraction spectrum, the peak intensity ratios, and the crystallite size of each MSE-type zeolite (with OSDA) in Examples and Comparative Examples are shown in Table 6.

**[Table 6]**

| | Organic structure directing agent (OSDA) | Peak intensity A (420) plane | | Peak intensity B (115) plane | | Peak intensity ratio (A / B) | Peak intensity ratio (A / α) | Crystallite size (nm) |
|---|---|---|---|---|---|---|---|---|
| | | 2θ = 21.0° - 22.0° | | 2θ = 22.6° - 23.6° | | | | |
| | | 2θ (deg) | Intensity A (cps) | 2θ (deg) | intensity B (cps) | | | |
| Example 1 | containing | 21.68 | 44700.0 | 22.88 | 25216.7 | 1.77 | 0.70 | 56.0 |
| Example 2 | containing | 21.66 | 48888.3 | 22.88 | 25000.0 | 1.96 | 0.77 | 72.8 |
| Example 3 | containing | 21.72 | 57133.3 | 22.90 | 31033.3 | 1.84 | 0.90 | 60.4 |
| Comparative Example 1 | containing | 21.66 | 31716.7 | 22.90 | 25500.0 | 1.24 | 0.50 | 32.3 |

The peak intensity at each diffraction angle (2θ) obtained from the X-ray diffraction spectrum, the peak intensity ratios, and the crystallite size of each MSE-type zeolite (without OSDA) in Examples and Comparative Examples are shown in Table 7.

**[Table 7]**

| | Organic structure directing agent (OSDA) | Peak intensity A (420) plane | | Peak intensity 8 (115) plane | | Peak intensity C (200) plane | | Peak intensity ratio (A / B) | Peak intensity ratio (A / α) | Peak intensity ratio (C / α) | Crystallite size (nm) |
|---|---|---|---|---|---|---|---|---|---|---|---|
| | | 2θ = 21.0° - 22.0° | | 2θ = 22.6° - 23.6° | | 2θ = 9.2° - 10.2° | | | | | |
| | | 2θ (deg) | Intensity A (cps) | 2θ (deg) | Intensity 8 (cps) | 2θ (deg) | intensity C (cps) | | | | |
| Example 1 | not containing | 21.70 | 20716.7 | 23.12 | 16233.3 | 9.68 | 13616.7 | 1.28 | 0.32 | 0.21 | 42.5 |
| Example 2 | not containing | 21.72 | 28383.3 | 23.10 | 19350.0 | 9.70 | 22850.0 | 1.47 | 0.45 | 0.36 | 56.2 |
| Example 3 | not containing | 21.74 | 35100.0 | 23.14 | 23116.7 | 9.72 | 23833.3 | 1.52 | 0.55 | 0.37 | 55.8 |
| Comparative Example 1 | not containing | 21.78 | 16500.0 | 23.12 | 17366.7 | 9.78 | 13000.0 | 0.95 | 0.26 | 0.20 | 22.1 |

The MSE-type zeolites (with OSDA) and the MSE-type zeolites (without OSDA) in Examples 1 to 3 had an X-ray diffraction spectrum with a peak intensity ratio (A/B) in the range of 1.25 or more and 2.50 or less, and had a low content of crystal phases other than the MSE-type crystal phase causing defects.

The MSE-type zeolites (with OSDA) in Examples 1 to 3 had a peak intensity ratio (A/α) in the range of 0.55 or more and 0.95 or less, contained a large amount of the MSE-type crystal phase, and had a low content of crystal phases other than the MSE-type crystal phase causing defects. The MSE-type zeolites (without OSDA) in Examples 1 to 3 had a peak intensity ratio (A/α) in the range of 0.28 or more and 0.60 or less, contained a large amount of the MSE-type crystal phase, and had a low content of crystal phases other than the MSE-type crystal phase causing defects.

The MSE-type zeolites (without OSDA) in Examples 1 to 3 had a peak intensity ratio (C/α) in the range of 0.15 or more and 0.50 or less, contained a large amount of the MSE-type crystal phase, and had a low content of crystal phases other than the MSE-type crystal phase causing defects.

The MSE-type zeolite (without OSDA) in Comparative Example 1 had a peak intensity ratio (A/B) of less than 1.0, and contained a large amount of crystal phases other than the MSE-type crystal phase. The MSE-type zeolite (with OSDA) in Comparative Example 1 had a peak intensity ratio (A/α) of less than 0.55. The MSE-type zeolite (without OSDA) in Comparative Example 1 had a peak intensity ratio (A/α) of less than 0.28.

FIGS. 5 and 6 show the X-ray diffraction spectrum of the MSE-type zeolite (with OSDA) in Example 3 and Comparative Example 1, respectively. The peak intensity A of the MSE-type zeolite (with OSDA) in Example 3 shown in FIG. 5 was higher compared to the peak intensity B, whereas the peak intensity A of the MSE-type zeolite (with OSDA) in Comparative Example 1 shown in FIG. 6 was lower compared to the peak intensity B.

From the X-ray diffraction spectrum of the MSE-type zeolite (with OSDA) in Example 3 shown in FIG. 5, no noticeable large peak of crystal phases other than the MSE-type crystal phase was confirmed, whereas from the X-ray diffraction spectrum of the MSE-type zeolite (with OSDA) in Comparative Example 1 shown in FIG. 6, a peak of crystal phases other than the MSE-type crystal phase indicating the (310) plane of an MTW-type zeolite was confirmed in the diffraction angle (2θ) range of 20° to 21°.

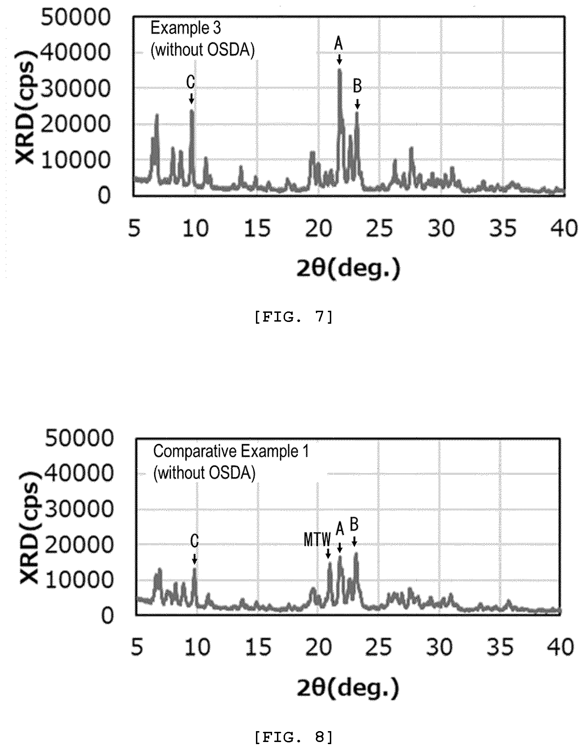

FIGS. 7 and 8 show the X-ray diffraction spectrum of the MSE-type zeolite (without OSDA) in Example 3 and Comparative Example 1, respectively. The peak intensity A of the MSE-type zeolite (without OSDA) in Example 3 shown in FIG. 7 was higher compared to the peak intensity B, whereas the peak intensity A of the MSE-type zeolite (without OSDA) in Comparative Example 1 shown in FIG. 8 was similar to the peak intensity B.

From the X-ray diffraction spectrum of the MSE-type zeolite (without OSDA) in Example 3 shown in FIG. 7, no noticeable large peak of crystal phases other than the MSE-type crystal phase was confirmed, whereas from the X-ray diffraction spectrum of the MSE-type zeolite (without OSDA) in Comparative Example 1 shown in FIG. 8, a peak of crystal phases other than the MSE-type crystal phase indicating the (310) plane of an MTW-type zeolite was confirmed in the diffraction angle (2θ) range of 20° to 21°.

### INDUSTRIAL APPLICABILITY

The pyrrolidine derivative according to the present invention can be used as an organic structure directing agent for producing a zeolite that contains few hardly-water-soluble impurities, easily forms an MSE-type crystal phase, and suppresses the formation of crystal phases other than the MSE-type crystal phase. The MSE-type zeolite produced by using the pyrrolidine derivative according to the present invention as an organic structure directing agent contains a large amount of the MSE-type crystal phase, has a low content of crystal phases other than the MSE-type crystal phase, which cause defects, and has high heat resistance, so that it can be suitably used as a hydrocarbon adsorbent for exhaust gas purifying catalysts.

## Claims

1. A pyrrolidine derivative comprising quaternary ammonium cations having a bicyclo framework, the pyrrolidine derivative having an X-ray diffraction spectrum with a highest peak intensity I in a diffraction angle (2θ) range of 14.0° to 16.0° and a highest peak intensity II in a diffraction angle (2θ) range of 17.0° to 19.0°, wherein a peak intensity ratio (I/II) of the peak intensity I to the peak intensity II is in a range of 0.4 or more and 1.5 or less.

2. The pyrrolidine derivative according to claim 1, having an X-ray diffraction spectrum of α-aluminum oxide with a peak intensity ratio (I/α) of the peak intensity I to a peak intensity α of the (116) plane in a range of 0.1 or more and 0.9 or less.

3. The pyrrolidine derivative according to claim 1 or 2, having an X-ray diffraction spectrum of α-aluminum oxide with a peak intensity ratio (II/α) of the peak intensity II to a peak intensity α of the (116) plane in a range of 0.3 or more and 0.5 or less.

4. The pyrrolidine derivative according to any one of claims 1 to 3, wherein a crystallite size determined from a peak having the peak intensity I is in a range of 45 nm or more and 120 nm or less.

5. The pyrrolidine derivative according to any one of claims 1 to 4, wherein the quaternary ammonium cations comprise N,N,N',N'-tetraalkylbicyclo[2.2.2]oct-7-ene-2,3:5,6-dipyrrolidinium dication and/or N,N,N',N'-tetraalkylbicyclo[2.2.2]octane-2,3:5,6-dipyrrolidinium dication.

6. An MSE-type zeolite having an X-ray diffraction spectrum with a highest peak intensity A in a diffraction angle (2θ) range of 21.0° to 22.0° representing the (420) plane, and a highest peak intensity B in a diffraction angle (2θ) range of 22.6° to 23.6° representing the (115) plane, wherein a peak intensity ratio (A/B) of the peak intensity A to the peak intensity B is in a range of 1.25 or more and 2.50 or less.

7. The MSE-type zeolite according to claim 8, wherein a crystallite size determined from a peak having the peak intensity A is in a range of 20 nm or more and 80 nm or less.

8. The MSE-type zeolite according to claim 6 or 7, comprising an organic structure directing agent, the MSE-type zeolite having an X-ray diffraction spectrum of α-aluminum oxide with a peak intensity ratio (A/α) of the peak intensity A to a peak intensity α of the (116) plane in a range of 0.55 or more and 0.95 or less.

9. The MSE-type zeolite according to claim 6 or 7, comprising no organic structure directing agent, the MSE-type zeolite having an X-ray diffraction spectrum of α-aluminum oxide with a peak intensity ratio (A/α) of the peak intensity A to a peak intensity α of the (116) plane in a range of 0.28 or more and 0.60 or less.

10. The MSE-type zeolite according to claim 9, having an X-ray diffraction spectrum with a peak intensity ratio (C/α) of a highest peak intensity C in a diffraction angle (2θ) range of 9.2° to 10.2° representing the (200) plane to the peak intensity α in a range of 0.15 or more and 0.50 or less.

11. A method for producing a pyrrolidine derivative comprising:
a first step of preparing N,N'-dialkylbicyclo[2.2.2]oct-7-ene-2,3;5,6-tetracarboxydiimide or N,N'-dialkylbicyclo[2.2.2]octane-2,3;5,6-tetracarboxydiimide;
a second step of reacting the N,N'-dialkylbicyclo[2.2.2]oct-7-ene-2,3;5,6-tetracarboxydiimide or the N,N'-dialkylbicyclo[2.2.2]octane-2,3;5,6-tetracarboxydiimide with a reducing agent to obtain N,N'-dialkylbicyclo[2.2.2]oct-7-ene-2,3;5,6-dipyrrolidine and/or N,N'-dialkylbicyclo[2.2.2]octane-2,3;5,6-dipyrrolidine; and
a third step of reacting the N,N'-dialkylbicyclo[2.2.2]oct-7-ene-2,3;5,6-dipyrrolidine and/or the N,N'-dialkylbicyclo[2.2.2]octane-2,3;5,6-dipyrrolidine with an alkyl halide to obtain an N,N,N',N'-tetraalkylbicyclo[2.2.2]oct-7-ene-2,3;5,6-dipyrrolidinium salt and/or an N,N,N',N'-tetraalkylbicyclo[2.2.2]octane-2,3;5,6-dipyrrolidinium salt,
wherein the method satisfies at least one of the following condition (1) or (2),
the condition (1) being that in the second step, the reducing agent comprises at least one selected from the group consisting of sodium bis(2-methoxyethoxy)aluminum hydride, lithium triethylborohydride, diisobutylaluminum hydride, diborane, lithium borohydride, and sodium borohydride, and
the condition (2) being that in the third step, the alkyl halide is reacted in the presence of an organic solvent in a molar ratio of 13 times or less relative to the N,N'-dialkylbicyclo[2.2.2]oct-7-ene-2,3;5,6-dipyrrolidine and/or the N,N'-dialkylbicyclo[2.2.2]octane-2,3;5,6-dipyrrolidine.

12. A method for producing an MSE-type zeolite comprising:
a step of preparing a raw material mixture containing a silica source, an aluminum source, an alkali metal source, the pyrrolidine derivative according to any one of claims 1 to 5, and water; and
a step of heating and crystallizing the raw material mixture.
